# EUROPÄISCHE PATENTSCHRIFT

(11) **EP 2 986 562 B1**
(45) Veröffentlichungstag und Bekanntmachung des Hinweises auf die Patenterteilung: **05.04.2017**
(21) Anmeldenummer: 14717751.3
(22) Anmeldetag: 14.04.2014
(51) Int. Cl.: C02F 1/02, C02F 1/20, C02F 9/00, C07C 201/08, C02F 1/04

(54) **VERFAHREN ZUR AUFARBEITUNG VON ABWASSER AUS DER NITRO-BENZOLHERSTELLUNG**
METHOD FOR THE TREATMENT OF WASTEWATER FROM THE PRODUCTION OF NITROBENZENE
PROCÉDÉ DE TRAITEMENT D'EAUX USÉES ISSUES DE LA FABRICATION DE NITROBENZÈNE

(30) Priorität: 18.04.2013 EP 13164307
(43) Veröffentlichungstag der Anmeldung: 24.02.2016
(73) Patentinhaber: Covestro Deutschland AG, 51373 Leverkusen (DE)
(72) Erfinder: KNAUF, Thomas, 41542 Dormagen (DE); MAIRATA, Antoni, 40549 Düsseldorf (DE); SLUYTS, Erik, B-2930 Brasschaat (BE); VAN TRICHT, Jan, B-2180 Ekeren (BE)
(74) Vertreter: Levpat
(86) Internationale Anmeldenummer: PCT/EP2014/057488
(87) Internationale Veröffentlichungsnummer: WO 2014/170250

(56) Entgegenhaltungen:
- EP-A1- 1 593 654
- WO-A1-2012/025393
- US-A1- 2012 078 015
- "Nitrobenzene fact sheet: support document", OPPT Chemical Fact Sheets, 1. Februar 1995 (1995-02-01), XP055081053, Gefunden im Internet: URL:http://www.epa.gov/chemfact/nitro-sd.p df [gefunden am 2013-09-25]

## Beschreibung

Die Erfindung betrifft ein Verfahren zur Aufarbeitung von alkalischem Abwasser, das bei der Wäsche von rohem, durch Nitrierung von Benzol erhaltenem Nitrobenzol entsteht, wobei
(i) das alkalische Abwasser unter Ausschluss von Sauerstoff bei einem gegenüber Atmosphärendruck erhöhten Druck erhitzt und anschließend abgekühlt und entspannt wird;
(ii) das in (i) erhaltene Abwasser durch Strippung mit einem Stripp-Gas weiter gereinigt und anschließend der mit Verunreinigungen beladene Stripp-Gasstrom auf eine Temperatur von 10 °C bis 60 °C abgekühlt wird;
(iii) das in (ii) durch Abkühlung des mit Verunreinigungen beladenen Stripp-Crasstroms erhaltene flüssige Verfahrensprodukt in eine wässrige und eine organische Phase getrennt wird und die organische Phase in einem Anilinproduktionsprozess weiterverwendet wird.

Nitrobenzol ist ein wichtiges Zwischenprodukt der chemischen Industrie, das insbesondere zur Herstellung von Anilin und damit auch zur Herstellung der Di- und Polyisocyanate der Diphenylmethanreihe und den darauf basierenden Polyurethanen benötigt wird.

Die Nitrierung von Benzol mit Salpetersäure zu einem Roh-Nitrobenzol war bereits Gegenstand zahlreicher Veröffentlichungen und Patentanmeldungen. Die heute gängigen Verfahren entsprechen im Wesentlichen dem Konzept der adiabaten Nitrierung von Benzol durch ein Gemisch von Schwefel- und Salpetersäure (sog. *Milchsäure*)*.* Ein solches Verfahren wurde erstmals in US 2,256,999 beansprucht und ist in heutigen Ausführungsformen beispielsweise in EP 0 436 443 B1**,** EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen.

Auch sind isotherme Verfahren zur Nitrierung von Benzol mit Mischsäure beschrieben, wie beispielsweise in EP 0 156 199 B1 beschrieben.

Auch sind Verfahren zur Nitrierung von Benzol bekannt, die ohne den Einsatz von Schwefelsäure auskommen. Diese sind beispielsweise beschrieben in US 2 739 174 oder US 3 780 116**.**

Grundsätzlich sind auch Gasphasenverfahren zur Nitrierung von Benzol mit Salpetersäure oder Stickstoffoxiden möglich, jedoch sind die dadurch erzielbaren Ausbeuten noch gering **(**EP 0 078 247 B1**,** EP 0 552 130 B1**).**

All diesen Verfahren ist gemeinsam, dass als Reaktionsprodukt zunächst ein Roh-Nitrobenzol entsteht, das als Verunreinigungen Salpetersäure und - sofern mit Mischsäure nitriert wurde - Schwefelsäure enthält, sowie als organische Verunreinigungen Dinitrobenzol sowie nitrierte Oxidationsprodukte des Benzols, insbesondere nitrierte Phenole (Nitrophenole). Auch enthält es organische Verbindungen, die aus den Verbindungen entstehen, die als Verunreinigungen im eingesetzten Benzol enthalten sind (WO 2008/148608 A1). Darüber hinaus enthält das Roh-Nitrobenzol auch Metallsalze, die in den Säureresten oder im Roh-Nitrobenzol gelöst vorliegen können (DE 10 2007 059 513 A1).

Zahlreiche Untersuchungen zielten in der Vergangenheit darauf ab die Qualität des Roh-Nitrobenzols zu verbessern und somit die Ausbeute bezüglich Benzol und Salpetersäure zu erhöhen. Dank dieser Entwicklungen sind die heutigen adiabaten Flüssigphasenverfahren so ausgereift, dass es allen gelingt, ein Roh-Nitrobenzol herzustellen, das einen geringen Gehalt an Nebenprodukten aufweist, also im Allgemeinen nur zwischen 100 ppm und 300 ppm an Dinitrobenzol und zwischen 1500 ppm und 2500 ppm an Nitrophenolen enthält, wobei Pikrinsäure einen Anteil von 10 % bis 50 % an den Nitrophenolen annehmen kann.

Das Roh-Nitrobenzol weist als Verunreinigungen noch Wasser, Benzol sowie Nitrophenole und Dinitrobenzol und - sofern mit Mischsäure nitriert wurde - Schwefelsäure auf. Diese Verunreinigungen sind unerwünscht, da sie in Nachfolgeprozessen, wo Nitrobenzol zum Einsatz kommt, wie zum Beispiel der Herstellung von Anilin, diese negativ beeinflussen können. Geeignete Aufarbeitungsverfahren, die Wasch- und Destillationsstufen beinhalten, sind z. B. in US 6,288,289 B1, EP 1 593 654 A1, EP 1 816 117 B1 und WO2011/021057 A1 beschrieben.

In EP 1 816 117B1 wird die Aufarbeitung des Roh-Nitrobenzols in einer sauren Wäsche, einer alkalischen Wäsche mit wässriger Natronlauge, einer neutralen Wäsche und einer abschließenden destillativen Reinigung beschrieben. Natürlich können grundsätzlich auch andere Basen als Natronlauge, wie zum Beispiel wässrige Natriumcarbonatlösung oder wässrige Ammoniaklösung (WO 2011/082 977 A1) oder Kaliumhydroxid oder Ammoniak (DE 60 113 579 T2) verwendet werden.

Die Aufarbeitung des alkalischen Abwassers aus der alkalischen Wäsche kann z. B. durch thermische Druckzersetzung (TDZ) erfolgen. Das grundsätzliche Verfahren der TDZ zur Behandlung von aromatischen Nitroverbindungen enthaltenden Abwässern ist in folgenden Patenten beschrieben:
In EP 0 005 203 B1 ist ein Verfahren beschrieben zur Aufarbeitung von Nitrohydroxyaromaten enthaltenden Abwässern, wobei die Abwässer unter Luft- und Sauerstoffausschluss bei einem Druck von 50 bar bis 250 bar und mit einer Temperatur von 150 °C bis 500 °C behandelt werden.

In EP 0 503 387 B1 wurde ein ähnliches Verfahren beschrieben, das aber dadurch gekennzeichnet ist, dass das besagte alkalische Abwasser durch Salpetersäurezugabe und anschließende Behandlung in Temperaturbereichen von 180 °C bis 350 °C und einem Druckbereich von 40 bar bis 250 bar aufgearbeitet wird. Beide Verfahren weisen jedoch erhebliche Nachteile auf:
EP 0 005 203 B1 beschreibt nicht die Entfernung von organischen Kohlenwasserstoffen wie Benzol oder Nitrobenzol, die in einem dem technischen Stand entsprechenden adiabaten Nitrierprozess anfallen. Die Reinigung des Abwassers gemäß der Lehre von EP 0 005 203 B1 ist daher entweder unzureichend, oder der Verbrauch an Natronlauge in der TDZ wird sehr hoch.

In EP 0 503 387 B1 gelingt nicht die vollständige Zersetzung von Nitrobenzol, so dass eine weitere Behandlung des Abwassers notwendig ist. Außerdem wird das im Abwasser enthaltene Nitrobenzol in der TDZ zersetzt und mindert daher die erzielte Ausbeute. Die nach der Lehre von EP 0 503 387 B1 erforderliche Anwesenheit von Salpetersäure in der TDZ treibt zudem die Prozesskosten in zweierlei Hinsicht in die Höhe: zum einen durch den Verbrauch an Salpetersäure und zum anderen durch die hohen Materialbelastungen infolge der Korrosivität der Salpetersäure und die damit verbunden hohen Investitionskosten, etwa für einen korrosionsbeständigen mit Titan ausgekleideten Rohrreaktor. Ein zusätzlicher, nicht beschriebener Nachteil ist auch die Notwendigkeit, dass das alkalische Abwasser zunächst einmal neutralisiert werden muss (z. B. mit zusätzlicher Salpetersäure), ehe dieses durch Zugabe der Salpetersäure in einen sauren pH-Bereich überführt werden kann. Es ist also eine verhältnismäßig große Menge an Säure erforderlich.

EP 1 593 654 A1 beschreibt ein Verfahren zur Aufarbeitung von alkalischen Abwässern, die bei der Wäsche von rohem Nitrobenzol entstehen, wobei das rohe Nitrobenzol durch adiabate Nitrierung von Benzol mit Nitriersäure hergestellt und anschließend in einer sauren Wäsche und danach in einer alkalischen Wäsche gewaschen wird, wobei ein alkalisches Abwasser enthaltend Benzol in Konzentrationen von 100 ppm bis 3000 ppm und Nitrobenzol in Konzentrationen von 1000 ppm bis 10000 ppm erhalten wird, wobei aus dem alkalischen Abwasser anschließend nicht gelöst vorliegendes Benzol und/oder Nitrobenzol abgeschieden wird, und optional aus dem alkalischen Abwasser anschließend restliches Benzol und/oder Nitrobenzol durch Strippen entfernt wird, und das alkalische Abwasser anschließend unter Ausschluss von Sauerstoff auf Temperaturen von 150 °C bis 500 °C unter Überdruck erhitzt wird. Das solchermaßen behandelte Abwasser kann daher ohne Verdünnung direkt einer biologischen Kläranlage zugeführt werden.

EP 0 953 546 B1 beschreibt ein Verfahren zum Abbau einer aromatischen Nitroverbindung oder eines Gemisches aus zwei oder mehr davon in Abwässern, die einen pH-Wert von 7 bis 14 aufweisen, bei dem die Abwässer auf Temperaturen von 150 °C bis 350 °C unter einem Druck von 10 bis 300 bar erhitzt werden, wobei mindestens eine aromatische Nitroverbindung keine Hydroxygruppe am aromatischen Ring trägt. Beispiele für solche aromatischen Nitroverbindungen sind Nitrohydroxyaromaten wie Mono-, Di- und Trinitrophenole, Mono-, Di-, Trinitrokresole, Mono-, Di- und Trinitroresorcine und Mono- Di-, Trixylenole. Aromatische Nitroverbindungen, die keine Hydroxygruppe am aromatischen Ring tragen, sind beispielsweise Nitrobenzole, Nitrotoluole und Dinitrotoluole. Die EP 0 953 546 B1 führt aus, dass die so behandelten Abwässer problemlos biologisch gereinigt werden können.

In WO2012/025393 A1 wird die Aufarbeitung von Abwässern, die bei der Aufreinigung von rohen aromatischen Nitroverbindungen nach der Nitrierung von aromatischen Verbindungen erhalten werden, beschrieben, und insbesondere die Problematik der Entfernung von Ammoniak, der bei der thermischen Zersetzung von Nitroverbindungen entsteht, behandelt. Das beschriebene Verfahren sieht folgende Schritte vor: (a) Einstufiges oder mehrstufiges Waschen der rohen aromatischen Nitroverbindung unter Erhalt mindestens einer organischen Phase und mindestens einer wässrigen Phase sowie Abtrennung der wässrigen Phase oder der wässrigen Phasen, wobei Schritt (a) die Zugabe einer Base umfasst, die sich von Ammoniak unterscheidet, und anschließend (b) optional Entfernung organischer Bestandteile aus mindestens einem Teil der in Schritt (a) erhaltenen wässrigen Phase oder wässrigen Phasen durch Strippung vorzugsweise mit Wasserdampf, anschließend (c) Entfernung organischer Verbindungen aus mindestens einem Teil der aus Schritt (a) beziehungsweise Schritt (b) resultierenden wässrigen Phase oder wässrigen Phasen durch thermischen und/oder oxidativen Abbau, anschließend (d) destillative Abreicherung von Ammoniak aus zumindest einem Teil der aus Schritt (c) resultierenden wässrigen Phase oder wässrigen Phasen, und anschließend (e) optional Zuführung zumindest eines Teiles der aus Schritt (d) resultierenden wässrigen Phase oder wässrigen Phasen zu einer biologischen Abwasseraufbereitung. Insbesondere sollte der Ammoniakgehalt im Abwasser in Schritt (d), welches bei der Nitrierung von aromatischen Verbindungen und anschließender Entfernung organischer Bestandteile anfällt, reduziert werden. In diesem Zusammenhang spricht WO 2012/025393 A1 von freiem Ammoniak und nicht von gelösten Ammoniumionen im Abwasser nach der TDZ, das einer biologischen Abwasseraufbereitung zugeführt wird. Aus dem im alkalischen Abwasser vorhandenen Kohlendioxid und Ammoniak bildet sich nämlich immer auch zu einem gewissen Teil Ammoniumcarbonat, das infolge seines Salzcharakters nicht durch Strippung entfernt werden kann wie Ammoniak.

Es bestand daher ein Bedarf an weiteren Verbesserungen des Verfahrens zur Aufarbeitung von alkalischen Abwässern, die bei der Wäsche von durch adiabate Nitrierung von Benzol hergestelltem rohem Nitrobenzol entstehen. Insbesondere sollte das Verfahren einfach und wirtschaftlich sein sowie eine schonende Behandlung der mitgeschleppten Organika beinhalten, sodass diese nicht zersetzt, sondern aus dem Abwasser zurückgewonnen werden können, bevor dieses einer Abwasseraufbereitung zugeführt wird.

Diesem Bedarf Rechnung tragend ist ein Gegenstand der vorliegenden Erfindung ein Verfahren zur Aufarbeitung von alkalischem Abwasser, das bei der Wäsche von rohem, durch - bevorzugt adiabate - Nitrierung von Benzol erhaltenem Nitrobenzol entsteht, wobei
(i) das alkalische Abwasser unter Ausschluss von Sauerstoff bei einem gegenüber Atmosphärendruck erhöhten Druck, bevorzugt bei einem absoluten Druck von 50 bar bis 350 bar, besonders bevorzugt von 50 bar bis 200 bar, ganz besonders bevorzugt von 70 bar bis 130 bar, auf eine Temperatur von 150 °C bis 500 °C, bevorzugt von 250 °C bis 350 °C, besonders bevorzugt von 270 °C bis 290 °C, für bevorzugt einen Zeitraum von 5 Minuten bis 120 Minuten, besonders bevorzugt von 15 Minuten bis 30 Minuten, erhitzt und anschließend bevorzugt auf eine Temperatur von 60 °C bis 100 °C abgekühlt und entspannt wird;
(ii) das in (i) erhaltene Abwasser durch Strippung mit einem Stripp-Gas, bevorzugt Wasserdampf, bevorzugt bei einem absoluten Druck von 0,1 bar bis 5 bar, besonders bevorzugt 0,5 bar bis 2 bar und bevorzugt bei einer Temperatur von 40 °C bis 160 °C, besonders bevorzugt 80 °C bis 120 °C, weiter gereinigt und anschließend der mit Verunreinigungen beladene Stripp-Gasstrom auf eine Temperatur von 10 °C bis 60 °C, bevorzugt von 20 °C bis 50 °C, besonders bevorzugt von 25 °C bis 45 °C, ganz besonders bevorzugt von 30 °C bis 40 °C, abgekühlt wird;
(iii) das in (ii) durch Abkühlung des mit Verunreinigungen beladenen Stripp-Gasstroms erhaltene flüssige Verfahrensprodukt in eine wässrige und eine organische Phase getrennt wird und die organische Phase in einem Anilinproduktionsprozess weiterverwenden wird.

Überraschend wurde nämlich gefunden, dass in dem erfindungsgemäßen Verfahren das Nitrobenzol, das mit dem alkalischen Abwasser in Schritt (i) gelangt, dort zumindest teilweise zu Anilin umgesetzt wird, sodass die erhaltene organische Phase vorteilhaft einem Anilinproduktionsprozess zugeführt werden kann.

Nachfolgend werden Ausführungsformen der Erfindung näher beschrieben. Dabei sind verschiedene Ausführungsformen beliebig miteinander kombinierbar, sofern sich aus dem Kontext nicht eindeutig das Gegenteil ergibt.

In einer besonders bevorzugten Ausführungsform umfasst der Gesamtprozess die folgenden Schritte:
a) Nitrierung von Benzol mit Salpetersäure oder - bevorzugt - einer Mischung aus Salpetersäure und Schwefelsäure (nachfolgend auch Mischsäure genannt) und Abtrennung der wässrigen Phase;
b) Wäsche des in Schritt a) erhaltenen organischen Verfahrensprodukts;
c) alkalische Wäsche des in Schritt b) erhaltenen gewaschenen organischen Verfahrensprodukts, wobei bevorzugt ein alkalisches Abwasser enthaltend Benzol in einer Konzentration von 100 ppm bis 3.000 ppm und Nitrobenzol in einer Konzentration von 1.000 ppm bis 10.000 ppm erhalten wird;
d) optionale Abscheidung von Benzol und/oder Nitrobenzol aus dem in Schritt c) erhaltenen alkalischem Abwasser;
e) Aufarbeitung des in Schritt c) oder Schritt d) erhaltenen alkalischen Abwassers umfassend die oben bezeichneten Schritte (i) bis (iii).

Die Nitrierung von Benzol zu Nitrobenzol mit Salpetersäure oder einem Gemisch aus Salpetersäure und Schwefelsäure (Mischsäure) in **Schritt a)** erfolgt dabei nach einem beliebigen der dem Fachmann bekannten Verfahren aus dem Stand der Technik, wie z. B. in EP 0 436 443 B1, EP 0 771 783 B1, US 6 562 247 B2 oder in EP 0 1.56 199 B1 beschrieben. Da in allen Verfahren des Standes der Technik ein Roh-Nitrobenzol erhalten wird, das überschüssige Säure, nicht umgesetztes Benzol, Wasser und organische Nebenkomponenten enthält, ist die erfindungsgemäße Reinigung des in Schritt a) erhaltenen Roh-Nitrobenzols grundsätzlich auf alle Verfahren anwendbar. Z. B. kann die Nitrierung unter Abfuhr der Reaktionswärme (d. h. isotherm oder angenähert isotherm) oder auch ohne Abfuhr der Reaktionswärme in bevorzugt isolierten Reaktoren (d. h. adiabat) erfolgen. Bevorzugt ist jedoch die Umsetzung von Benzol mit einem Gemisch aus Salpetersäure und Schwefelsäure unter adiabater Verfahrensführung, wie sie insbesondere in DE 10 2008 048 713 A1, und dort insbesondere in Absatz [0024], beschrieben ist. Das in Schritt a) hergestellte rohe Nitrobenzol wird schließlich in einem Trennbehälter von überschüssiger Säure (bei Verwendung von Mischsäure im Wesentlichen Schwefelsäure) getrennt.

Die in Schritt a) nach der Phasentrennung erhaltene organische Phase, die üblicherweise noch Spuren an Säure enthält, wird in **Schritt b)** in einer bis zwei, bevorzugt einer Wäsche(n) gewaschen und dann von der sauren wässrigen Phase durch Phasentrennung getrennt (bei mehreren Wäschen nach jeder einzelnen Wäsche). In Schritt b) werden die Säurereste, die das rohe Nitrobenzol enthält, ausgewaschen; daher wird dieser Verfahrensschritt auch als *saure Wäsche* bezeichnet. Bevorzugt wird dabei so verfahren, dass sich in der nach der Phasentrennung erhaltenen wässrigen Phase ein pH-Wert < 5 (gemessen bei 20 °C) eingestellt. Als Waschflüssigkeit kann in Schritt b) jede Art von Wasser, z. B. vollentsalztes Wasser oder Dampfkondensat, eingesetzt werden. Das Wasser kann auch gelöste Salze enthalten. Bevorzugt werden zur Durchführung von Schritt b) im Betrieb anfallende wässrige Ströme rezyklisiert.

Die so erhaltene organische Phase wird anschließend in **Schritt c)** in bevorzugt einer bis zwei, besonders bevorzugt einer alkalischen Wäsche(n) mit einer Base, bevorzugt einer wässrigen Lösung einer Base ausgewählt aus der Gruppe bestehend aus Natriumhydroxid, Natriumcarbonat und Natriumhydrogencarbonat, gewaschen und anschließend von dem alkalischen Waschwasser durch Phasentrennung getrennt (bei mehreren Wäschen nach jeder einzelnen Wäsche). Bevorzugt wird als wässrige Baselösung Natronlauge eingesetzt. Die alkalische Wäsche wird im Folgenden anhand von Natronlauge beschrieben; für den Fachmann ist es ein Leichtes, bei Einsatz anderer Basen erforderlichenfalls entsprechende Abänderungen vorzunehmen.

Bevorzugt hat die eingesetzte Natronlauge einen pH-Wert von 9,0 bis 14 (gemessen bei 20 °C). Das Massenverhältnis von Natronlauge zu organischer Phase (im Wesentlichen Nitrobenzol) ist abhängig vom in Schritt a) eingesetzten Benzolüberschuss und liegt bevorzugt zwischen 1 : 80 und 1 : 500. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt b) nicht vollständig entfernte Säurereste) in Schritt c) weitgehend bis vollständig, bevorzugt vollständig, neutralisiert werden. Bevorzugt wird die Base in Schritt c) in einem Überschuss von 0,5 % bis 5,0 % der Theorie, besonders bevorzugt von 1,0 % bis 3,0 % der Theorie und ganz besonders bevorzugt von 1,5 % bis 2,5 % der Theorie, jeweils bezogen auf die im Abwasser nach Schritt b) enthaltenen Nitrophenole, eingesetzt. Dabei ist der maßgebliche Wert für den Nitrophenolgehalt der durch Gaschromatographie bestimmte.

Im optionalen **Schritt d)** wird aus dem alkalischen Abwasser aus Schritt c) noch enthaltenes, nicht gelöst vorliegendes Benzol und/oder Nitrobenzol abgetrennt. Das so abgetrennte Benzol und/oder Nitrobenzol wird dann bevorzugt wieder dem Nitrierprozess, besonders bevorzugt dem rohen Nitrobenzol, zugeführt. Die Abtrennung des nicht gelöst vorliegenden Nitrobenzols kann dabei durch Abscheider, Absetzbehälter oder andere Phasentrennapparaturen erfolgen. Bevorzugt wird ein Absetzbehälter eingesetzt. Bevorzugt wird in Schritt d) ein alkalisches Abwasser erhalten, das Benzol in einer Konzentration von 100 ppm bis 1000 ppm und Nitrobenzol in einer Konzentration von 1200 ppm bis 3000 ppm enthält. Es ist bevorzugt, Schritt d) durchzuführen.

In **Schritt e) (i)** (der TDZ) wird das aus den Schritten c bzw. d) erhaltene alkalische Abwasser, das noch mit organischen Salzen der Nitrohydroxyaromaten beladen ist, unter Ausschluss von Sauerstoff bei einem gegenüber Atmosphärendruck erhöhten Druck, bevorzugt bei einem absoluten Druck von 50 bar bis 350 bar, besonders bevorzugt von 50 bar bis 200 bar, ganz besonders bevorzugt von 70 bar bis 130 bar, auf eine Temperatur von 150 °C bis 500 °C, bevorzugt von 250 °C bis 350 °C, besonders bevorzugt von 270 °C bis 290 °C, erhitzt. Es ist auch möglich, das alkalische Abwasser unter Inertgasatmosphäre bzw. unter einem Inertgas-Vordruck von beispielsweise 0,1 bar bis 100 bar zu erhitzen. Als Inertgase sind z. B. Stickstoff und/oder Argon geeignet. Je nach Temperatur und gegebenenfalls Inertgas-Vordruck stellen sich beim Erhitzen der Abwässer bevorzugt die zuvor genannten Drücke ein. Die Erhitzung des alkalischen Abwassers und thermische Druckzersetzung der organischen Bestandteile wie Benzol, Nitrobenzol und Nitrohydroxyaromaten erfolgt dabei üblicherweise für 5 Minuten bis 120 Minuten, bevorzugt 15 Minuten bis 30 Minuten. Bevorzugt wird das alkalische Abwasser anschließend so abgekühlt, dass es die TDZ mit einer Temperatur von 60 °C bis 100 °C verlässt. Die Abkühlung erfolgt bevorzugt im Gegenstrom mit dem Eingangsstrom unter Entspannung.

Die Schritte d) und e) (i) können gemäß dem Stand der Technik, bevorzugt gemäß der Offenbarung der EP 1 593 654 A1, durchgeführt werden.

Besonders bevorzugt ist es, die Verweilzeit in Schritt e (i) und den Überschuss an Base, der in Schritt c) eingesetzt wird, aufeinander abzustimmen. So ist bei einer kurzen Verweilzeit in Schritt e) (i) ein größerer Basenüberschuss in Schritt c) vorteilhaft.

**In Schritt e) (ii)** wird das in e) (i) erhaltene Abwasser durch Strippung weiter gereinigt und anschließend der mit Verunreinigungen beladene Stripp-Gasstrom auf eine Temperatur von 10 °C bis 60 °C, bevorzugt von 20 °C bis 50 °C, besonders bevorzugt von 25 °C bis 45 °C, ganz besonders bevorzugt von 30 °C bis 40 °C, abgekühlt. Hierbei werden noch vorhandene organische Bestandteile entfernt. Bevorzugt erfolgt die Strippung im Rahmen der vorliegenden Erfindung im Gegenstrom in einer Strippkolonne, wobei der Gasstrom aus dem eingesetzten Strippgas (bevorzugt Wasserdampf) und den abgestrippten leichtflüchtigen Bestandteilen bevorzugt am Kopf der Strippkolonne austritt (Brüden) und das gestrippte Abwasser bevorzugt am Sumpf der Strippkolonne entnommen wird. Der am Kopf der Strippkolonne entnommene Gasstrom (Brüden) wird in einem Kondensator auf eine Temperatur von 10 °C bis 60 °C, bevorzugt von 20 °C bis 50 °C, besonders bevorzugt von 25 °C bis 45 °C, ganz besonders bevorzugt von 30 °C bis 40 °C abgekühlt. Abgestrippter, gasförmig verbliebener Ammoniak wird bevorzugt einer technischen Abluftreinigung zugeführt.

Die Strippkolonne ist vorzugsweise eine röhrenförmige Einrichtung mit mehreren Einbauten (z. B. Schüttungen aus Füllkörpern, strukturierte Packungen oder Stoffaustauschböden) zum intensiven Stoffaustausch von gasförmiger und flüssiger Phase. Entsprechende Verfahren und Kolonnen sind dem Fachmann bekannt und z. B. in W. Meier, Sulzer, Kolonnen für Rektifikation und Absorption, in: Technische Rundschau Sulzer, 2 (1979), Seite 49 bis 61, beschrieben. Bevorzugt wird die Strippung gemäß Schritt e) (ii) bei einem absoluten Druck von 0,1 bar bis 5 bar, besonders bevorzugt 0,5 bar bis 2 bar, und bevorzugt bei einer Temperatur von 40 °C bis 160 °C, besonders bevorzugt 80 °C bis 120 °C, durchgeführt.

**In Schritt e) (iii)** wird der in Schritt e) (ii) durch Abkühlung des Gasstroms aus dem eingesetzten Stripp-Gas (bevorzugt Wasserdampf) und den abgestrippten leichtflüchtigen Bestandteilen erhaltene flüssige Strom in eine wässrige und eine organische Phase getrennt. Dies geschieht in einem Phasentrennapparat, aus dem die sich abscheidenden Organika ausgeschleust werden. Der so erhaltene von Organika weitgehend befreite Strom (in der bevorzugten Ausführungsform mit Wasserdampf als Stripp-Gas, das sog. *Brüdenwasser*) wird bevorzugt teilweise bis vollständig auf den Kopf der Strippkolonne zurückgefahren. Der verbleibende, nicht auf den Kopf der Strippkolonne zurückgefahrene Teil des von Organika weitgehend befreiten Stroms wird bevorzugt direkt (ohne weitere zwischengeschaltete Reinigungsschritte) einer Abwasseraufbereitung, bevorzugt einer biologischen Kläranlage, zugeführt.

Erfindungsgemäß wird die in Schritt (iii) erhaltene aus dem Phasentrennapparat ausgeschleuste organische Phase einem Anilinproduktionsprozess zugeführt. Dabei handelt es sich bevorzugt um einen Prozess zur Herstellung von Anilin durch katalytische Hydrierung von Nitrobenzol. Die katalytische Hydrierung von Nitrobenzol erfolgt bevorzugt in der Gasphase unter Rückführung nicht umgesetzten Wasserstoffs (Kreisgasfahrweise). Die Zufuhr der organischen Phase aus Schritt (iii) kann an verschiedenen Stellen erfolgen:
So ist es möglich, die organische Phase aus Schritt (iii) einem Eduktstrom einer Anilinproduktionsanlage zuzuführen. Bspw. enthält im großtechnischen Betrieb unter Rückführung nicht umgesetzten Wasserstoffs der Einsatzwasserstoff, ein Gemisch aus frisch zugeführtem Wasserstoff und zurückgeführte Wasserstoff, ohnehin Anteile an nach erfolgter Reaktion nicht vollständig abgetrennten Anilins. Es ist natürlich auch möglich, die organische Phase aus Schritt (iii) dem Produktstrom der Anilinproduktionsanlage zuzuführen, bevorzugt nach Abkühlung desselben und vor der sich anschließenden destillativen Aufarbeitung des rohen Anilins.

### Beispiele

### Messmethoden

Gehalt an organischen Komponenten: Gaschromatographie (GC), angegeben sind Flächen-%.

Gehalt an Kationen: Atomabsorptionspektrometrie (Inductive Coupled Plasma, ICP), angegeben sind Massenanteile in ppm.

### Allgemeine Bedingungen für die Herstellung von Nitrobenzol

Nitrobenzol wurde in einem adiabaten Prozess wie in EP 2 168 942 A1 beschrieben hergestellt. Das dabei in der letzten alkalischen Wäsche anfallende Abwasser wurde in den nachfolgenden Beispielen verwendet.

### Beispiel 1 (erfindungsgemäß)

Das Abwasser aus der alkalischen Wäsche wurde in einen Absetztank gefahren, in dem ungelöstes Benzol und Nitrobenzol abgeschieden wurden. 3,5 Tonnen pro Stunde an alkalischem Abwasser, das einen Gehalt an Nitrobenzol von 2870 ppm, an Benzol von 409 ppm und an Nitrophenolen an 11809 ppm sowie einen pH-Wert von 12,8 hatte (1,8% NaOH-Überschuss gegenüber dem Ausgangsgehalt an Nitrophenolen vor der alkalischen Wäsche), wurden von dort bei einer Verweilzeit von 20 min, einer Temperatur von 290 °C und einem absoluten Druck von 90 bar behandelt. Das entstandene Abwasser wurde auf 80 °C abgekühlt (Schritt (i)). Der Umsatz an Nitrobenzol in der TDZ beträgt 99 %. Anschließend wurde das Abwasser mit Direktdampf gestrippt (Schritt (ii)). Die Strippkolonne wurde bei einem absoluten Druck von 1,05 bar betrieben. Im Sumpf der Strippkolonne wurde ein Strom von 3,9 Tonnen pro Stunde erhalten, der im Wesentlichen Wasser, Ammoniak und Organika enthielt. Das Kopfprodukt der Strippkolonne wurde kondensiert, in eine wässrige und eine organische Phase getrennt (Schritt (iii)), und 280 kg pro Stunde des wässrigen Stromes wurden als Rückfluss in die Kolonne zurückgeführt. Die untere organische Phase im Phasentrennapparat wurde zur Weiterverarbeitung in den Rohanilintank einer Anilinanlage gefahren. Die organische Phase enthielt pro Stunde 2,6 kg Anilin, 0,2 kg Nitrobenzol, 1,5 kg Benzol und 1 kg Phenol. Die Ausbeute an in der TDZ gewonnenem Anilin, bezogen auf Nitrobenzol, betrug 22 %.

### Beispiel 2 (erfindungsgemäß)

Das Abwasser aus der alkalischen Wäsche wurde in einen Absetztank gefahren, in dem ungelöstes Benzol und Nitrobenzol abgeschieden wurden. 3,5 Tonnen pro Stunde an alkalischem Abwasser, das einen Gehalt an Nitrobenzol von 2915 ppm, an Benzol von 382 ppm und an Nitrophenolen von 12051 ppm sowie einen pH-Wert von 13,4 hatte (2,3 % NaOH-Überschuss gegenüber dem Ausgangsgehalt an Nitrophenolen vor der alkalischen Wäsche), wurden bei einer Verweilzeit von 20 min, 290 °C und einem absoluten Druck von 90 bar behandelt. Das entstandene Abwasser wurde auf 80 °C abgekühlt (Schritt (i)). Der Umsatz an Nitrobenzol in der TDZ beträgt 99,9%. Anschließend wurde das Abwasser mit Direktdampf gestrippt. Die Strippkolonne wurde bei einem absoluten Druck von 1,05 bar betrieben. Im Sumpf der Strippkolonne wurde ein Strom von 3,9 Tonnen pro Stunde erhalten, der im Wesentlichen Wasser, Ammoniak und Organika enthielt. Das Kopfprodukt der Strippkolonne wurde kondensiert, in eine wässrige und eine organische Phase getrennt (Schritt (iii)), und 280 kg pro Stunde des wässrigen Stromes wurden als Rückfluss in die Kolonne zurückgeführt. Die untere organische Phase im Phasentrennapparat wird zur Weiterverarbeitung in den Rohanilintank einer Anilinanlage gefahren. Die organische Phase enthält pro Stunde 5,9 kg Anilin, 0,01 kg Nitrobenzol, 1,2 kg Benzol und 0,8 kg Phenol. Die Ausbeute an in der TDZ gewonnenem Anilin, bezogen auf Nitrobenzol, betrug 49 %.

### Beispiel 3 (erfindungsgemäß)

Das Abwasser aus der alkalischen Wäsche wurde in einen Absetztank gefahren, in dem ungelöstes Benzol und Nitrobenzol abgeschieden wurden. 3,6 Tonnen pro Stunde an alkalischem Abwasser, das einen Gehalt an Nitrobenzol von 2420 ppm, an Benzol von 220 ppm und an Nitrophenolen an 11234 ppm sowie einen pH-Wert von 13,0 hatte (1,8% NaOH-Überschuss gegenüber dem Ausgangsgehalt an Nitrophenolen vor der alkalischen Wäsche), wurden bei einer Verweilzeit von 41 min, 280 °C und einem absoluten Druck von 106 bar behandelt. Das entstandene Abwasser wurde anschließend auf 80 °C abgekühlt (Schritt (i)). Der Umsatz an Nitrobenzol in der TDZ beträgt 99,9 %. Anschließend wurde das Abwasser mit Direktdampf gestrippt. Die Strippkolonne wurde bei einem absoluten Druck von 1,03 bar betrieben (Schritt (ii)). Im Sumpf der Strippkolonne wurde ein Strom von 4,0 Tonnen pro Stunde erhalten, der im Wesentlichen Wasser, Ammoniak und Organika enthielt. Das Kopfprodukt der Strippkolonne wurde kondensiert, in eine wässrige und eine organische Phase getrennt (Schritt (iii)), und 680 kg pro Stunde des wässrigen Stromes wurden als Rückfluss in die Kolonne zurückgeführt. Die untere organische Phase im Phasentrennapparat wurde zur Weiterverarbeitung in den Rohanilintank einer Anilinanlage gefahren. Die organische Phase enthielt pro Stunde 10 kg Anilin, 0,04 kg Nitrobenzol, 0,01 kg Benzol und 0,02 kg Phenol. Die Ausbeute an in der TDZ gewonnenem Anilin, bezogen auf Nitrobenzol, betrug 99 %.

## Patentansprüche

1. Verfahren zur Aufarbeitung von alkalischem Abwasser, das bei der Wäsche von rohem, durch Nitrierung von Benzol erhaltenem Nitrobenzol entsteht, wobei
(i) das alkalische Abwasser unter Ausschluss von Sauerstoff bei einem gegenüber Atmosphärendruck erhöhten Druck auf eine Temperatur von 150 °C bis 500 °C erhitzt und anschließend abgekühlt und entspannt wird;
(ii) das in (i) erhaltene Abwasser durch Strippung mit einem Stripp-Gas weiter gereinigt und anschließend der mit Verunreinigungen beladene Stripp-Gasstrom auf eine Temperatur von 10 °C bis 60 °C abgekühlt wird;
(iii) das in (ii) durch Abkühlung des mit Verunreinigungen beladenen Stripp-Gasstroms erhaltene flüssige Verfahrensprodukt in eine wässrige und eine organische Phase getrennt wird und die organische Phase in einem Anilinproduktionsprozess weiterverwendet wird.

2. Verfahren nach Anspruch 1, bei dem die in Schritt (iii) erhaltene organische Phase dem Eduktstrom einer Anilinproduktionsanlage zugeführt wird.

3. Verfahren nach Anspruch 1, bei dem die in Schritt (iii) erhaltene organische Phase dem Produktstrom einer Anilinproduktionsanlage zugeführt wird.

4. Verfahren nach einem der Ansprüche 1 bis 3, bei dem das Erhitzen des alkalischen Abwassers in Schritt (i) bei einem absoluten Druck von 50 bar bis 350 bar durchgeführt wird.

5. Verfahren nach einem der Ansprüche 1 bis 4, bei dem das Erhitzen des alkalischen Abwassers in Schritt (i) für einen Zeitraum von 5 Minuten bis 120 Minuten durchgeführt wird.

6. Verfahren nach einem der Ansprüche 1 bis 4, bei dem das alkalische Abwasser nach dem Erhitzen auf eine Temperatur von 60 °C bis 100 °C abgekühlt wird.

7. Verfahren nach einem der Ansprüche 1 bis 6, bei dem die in Schritt (iii) erhaltene wässrige Phase teilweise in die Strippung von Schritt (ii) zurückgeführt und der verbleibende, nicht zurückgeführte Teil ohne weitere Reinigungsschritte einer biologischen Kläranlage zugeführt wird.

8. Verfahren nach einem der Ansprüche 1 bis 7, bei dem das in Schritt (i) eingesetzte alkalische Abwasser aus Schritt c) oder Schritt d) der folgenden Verfahrensschritte stammt:
a) Nitrierung von Benzol mit Salpetersäure oder einer Mischung aus Salpetersäure und Schwefelsäure und Abtrennung der wässrigen Phase;
b) Wäsche des in Schritt a) erhaltenen organischen Verfahrensprodukts;
c) alkalische Wäsche des in Schritt b) erhaltenen gewaschenen organischen Verfahrensprodukts;
d) optionale Abscheidung von Benzol und/oder Nitrobenzol aus dem in Schritt c) erhaltenem alkalischen Abwasser.

9. Verfahren nach Anspruch 8, bei dem in der alkalischen Wäsche in Schritt c) die Base in einem Überschuss von 0,5 % bis 5,0 % der Theorie, bezogen auf die im Abwasser nach Schritt b) enthaltenen Nitrophenole, eingesetzt wird.

## Claims

1. Process for working up alkaline waste water which is formed during washing of crude nitrobenzene obtained by nitration of benzene, wherein
(i) the alkaline waste water is heated to a temperature of from 150 °C to 500 °C under an increased pressure with respect to atmospheric pressure with exclusion of oxygen and is then cooled and depressurized;
(ii) the waste water obtained in (i) is purified further by stripping with a stripping gas and the stripping gas stream loaded with impurities is then cooled to a temperature of from 10 °C to 60 °C ;
(iii) the liquid process product obtained in (ii) by cooling the stripping gas stream loaded with impurities is separated into an aqueous and an organic phase and the organic phase is used further in an aniline production process.

2. Process according to claim 1, in which the organic phase obtained in step (iii) is sent to the reactant stream of an aniline production plant.

3. Process according to claim 1, in which the organic phase obtained in step (iii) is sent to the product stream of an aniline production plant.

4. Process according to one of claims 1 to 3, in which the heating of the alkaline waste water in step (i) is carried out under an absolute pressure of from 50 bar to 350 bar.

5. Process according to one of claims 1 to 4, in which the heating of the alkaline waste water in step (i) is carried out for a period of from 5 minutes to 120 minutes.

6. Process according to one of claims 1 to 4, in which after the heating the alkaline waste water is cooled to a temperature of from 60 °C to 100 °C.

7. Process according to one of claims 1 to 6, in which the aqueous phase obtained in step (iii) is partially recycled into the stripping of step (ii) and the remaining part which is not recycled is sent to a biological treatment plant without further purification steps.

8. Process according to one of claims 1 to 7, in which the alkaline waste water used in step (i) originates from step c) or step d) of the following process steps:
a) nitration of benzene with nitric acid or a mixture of nitric acid and sulfuric acid and separating off of the aqueous phase;
b) washing of the organic process s product
obtained in step a);
c) alkaline washing of the washed organic process product obtained in step b);
d) optional separation of benzene and/or nitrobenzene out of the alkaline waste water obtained in step c).

9. Process according to claim 8, in which in the alkaline wash in step c) the base is used in an excess of from 0.5 % to 5.0 % of theory, based on the nitrophenols contained in the waste water after step b).

## Revendications

1. Procédé de traitement d'une eau résiduaire alcaline, qui est formée lors du lavage de nitrobenzène brut obtenu par nitration de benzène, selon lequel
(i) l'eau résiduaire alcaline est portée à une température de 150 °C à 500 °C avec exclusion d'oxygène à une pression élevée par rapport à la pression atmosphérique, puis refroidie et détendue ;
(ii) l'eau résiduaire obtenue en (i) est davantage purifiée par extraction avec un gaz d'extraction, puis le courant de gaz d'extraction chargé avec des impuretés est refroidi à une température de 10 °C à 60 °C ;
(iii) le produit de procédé liquide obtenu en (ii) par refroidissement du courant de gaz d'extraction chargé avec des impuretés est séparé en une phase aqueuse et une phase organique, puis la phase organique est utilisée dans un procédé de production d'aniline.

2. Procédé selon la revendication 1, selon lequel la phase organique obtenue à l'étape (iii) est introduite dans le courant de réactifs d'une unité de production d'aniline.

3. Procédé selon la revendication 1, selon lequel la phase organique obtenue à l'étape (iii) est introduite dans le courant de produits d'une unité de production d'aniline.

4. Procédé selon l'une quelconque des revendications 1 à 3, selon lequel le chauffage de l'eau résiduaire alcaline à l'étape (i) est réalisé à une pression absolue de 50 bar à 350 bar.

5. Procédé selon l'une quelconque des revendications 1 à 4, selon lequel le chauffage de l'eau résiduaire alcaline à l'étape (i) est réalisé pendant une durée de 5 minutes à 120 minutes.

6. Procédé selon l'une quelconque des revendications 1 à 4, selon lequel l'eau résiduaire alcaline est refroidie à une température de 60 °C à 100 °C après le chauffage.

7. Procédé selon l'une quelconque des revendications 1 à 6, selon lequel la phase aqueuse obtenue à l'étape (iii) est recyclée en partie dans l'extraction de l'étape (ii), et la partie non recyclée restante est introduite sans étapes de purification supplémentaires dans une unité d'épuration biologique.

8. Procédé selon l'une quelconque des revendications 1 à 7, selon lequel l'eau résiduaire alcaline utilisée à l'étape (i) provient de l'étape c) ou de l'étape d) des étapes de procédé suivantes :
a) la nitration de benzène avec de l'acide nitrique ou un mélange d'acide nitrique et d'acide sulfurique, et la séparation de la phase aqueuse ;
b) le lavage du produit de procédé organique obtenu à l'étape a) ;
c) le lavage alcalin du produit de procédé organique lavé obtenu à l'étape b) ;
d) éventuellement la séparation de benzène et/ou de nitrobenzène de l'eau résiduaire alcaline obtenue à l'étape c).

9. Procédé selon la revendication 8, selon lequel la base est utilisée dans le lavage alcalin de l'étape c) en un excès de 0,5 % à 5,0 % de la théorie, par rapport aux nitrophénols contenus dans l'eau résiduaire après l'étape b).
